**Europäisches Patentamt**

**European Patent Office**

**Office européen des brevets**

(19)

(11) Veröffentlichungsnummer: **0 124 756**
**A2**

(12) # EUROPÄISCHE PATENTANMELDUNG

(21) Anmeldenummer: **84103542.1**

(22) Anmeldetag: **30.03.84**

(51) Int. Cl.³: **C 07 C 121/413**
**C 07 C 120/00**

(30) Priorität: **07.04.83 DE 3312426**

(43) Veröffentlichungstag der Anmeldung:
**14.11.84 Patentblatt 84/46**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Bruhn, Athanas, Dr.**
**Hauptstrasse 57**
**D-8870 Günzburg(DE)**

(72) Erfinder: **Harth, Hubert, Dr.**
**Buchenstrasse 31**
**D-4000 Düsseldorf 13(DE)**

(72) Erfinder: **Wüst, Willi, Dr.**
**Fasanenring 32**
**D-4030 Ratingen 6(DE)**

(54) **Verfahren zur Herstellung von 2-Cyanacrylaten.**

(57) Beschrieben wird ein Verfahren zur Herstellung von monomeren 2-Cyanacrylaten aus Cyanacetaten und Formaldehyd oder Polyoxymethylen, bei dem die Kondensation in Gegenwart von 5 bis 80 Gewischtsprozent Wasser stattfindet. Die Kondensationsprodukte werden nach Entwässern in üblicher Weise thermisch gespalten. Dabei werden monomere 2-Cyanacrylate in erhöhter Ausbeute und verbesserter Qualität erhalten.

EP 0 124 756 A2

Henkelstraße 67
4ooo Düsseldorf, den  5.4.1983

01247 56
HENKEL KGaA
ZR-FE/Patente

Dr.Wik/Po

P a t e n t a n m e l d u n g

D 6621 EP

"Verfahren zur Herstellung von 2-Cyanacrylaten"

Die Erfindung liegt auf dem Gebiet der Reaktionsklebstoffe. Sie betrifft ein neues, verbessertes Verfahren
zur Herstellung von monomeren 2-Cyanacrylaten.

2-Cyanacrylate sind seit vielen Jahren bekannt. Sie unterscheiden sich von den allermeisten bekannten ethylenisch ungesättigten Verbindungen dadurch, daß sie
aufgrund ihrer hohen Polarität bereits in Gegenwart
von Wasser zur anionischen Polymerisation befähigt sind.
Die Verbindungen werden deshalb als feuchtigkeitshärtende Reaktivklebstoffe für zahlreiche Anwendungen eingesetzt. Dabei werden mitunter beachtliche Festigkeiten
erzielt.

Es sind zahlreiche Verfahren bekannt, Ester der 2-Cyanacrylsäure - also 2-Cyanacrylate - herzustellen. So wird
bereits in der US-PS 2 467 927 vorgeschlagen, Cyanacetate in Formaldehydlösung einzutropfen. Dabei entsteht
eine viskose Masse, die sich aus dem wässrigen Reaktionsmedium abscheidet. Durch thermische Zersetzung dieses
hochmolekularen Zwischenproduktes sind dann die monomeren
2-Cyanacrylate herstellbar. Das Arbeiten nach dieser
Lehre führt jedoch zu unbefriedigenden Ergebnissen. Durch
den hochmolekularen Charakter des anfallenden Zwischen-

...

- 2 -

produktes ist dessen Verarbeitung, insbesondere die Trocknung nur mit aufwendigen technischen Mitteln möglich. Weiterhin weisen die erhaltenen Produkte nur geringe Lagerstabilität auf.

Es wurde daher in der deutschen Patentschrift 12 58 405 vorgeschlagen, statt in Wasser in einem organischen Lösungsmittel zu arbeiten und einen molaren Unterschuß an Polyoxymethylen (Paraformaldehyd) zu den entsprechenden Cyanacetaten zuzugeben. Nach der Lehre dieser Patentschrift sind 2-Cyanacrylate in verbesserter Qualität jedoch nach wie vor in nicht befriedigender Ausbeute herstellbar.

Ausgehend von dieser Lehre wurden in der Folgezeit zahlreiche Verbesserungen bekannt, doch galt in den hier betroffenen Fachkreisen in Anbetracht der hohen Wasserempfindlichkeit der Endprodukte das Arbeiten in aprotischen, organischen Lösungsmitteln als notwendig.

Aufgrund der Bedeutung der 2-Cyanacrylate bestand somit Bedarf nach einem neuen verbesserten Herstellungsverfahren für diese Produkte.

Aufgabe der Erfindung ist es somit ein Verfahren bereitzustellen, nach dem 2-Cyanacrylate - insbesondere die Ester der 2-Cyanacrylsäure mit $C_1$ - $C_8$ - Alkoholen - mit verbesserter Ausbeute hergestellt werden können. Eine weitere Aufgabe der Erfindung ist es, ein diesbezügliches Verfahren bereitzustellen, welches ohne die Mitverwendung organischer Lösungsmittel arbeitet.

...

Gegenstand der Erfindung ist somit ein Verfahren zur Herstellung von monomeren 2-Cyanacrylaten durch Umsetzung der entsprechenden Cyanessigsäureester mit einem molaren Unterschuß an Formaldehyd und/oder Polyoxymethylen bei erhöhter Temperatur und anschließender thermischer Spaltung des entstehenden Zwischenproduktes, dadurch gekennzeichnet, daß man die Umsetzung des Cyanacetats mit Formaldehyd und/oder Polyoxymethylen in Gegenwart von Wasser bei einem pH-Wert 3 - 7 oder 7 - 14, gegebenenfalls unter erhöhtem Druck durchführt.

Das erfindungsgemäße Verfahren macht sich zunächst die literaturbekannte Tatsache zunutze, daß bei der Umsetzung von Cyanacetaten mit Formaldehyd oder Polyoxymethylen nur dann niederviskose, gut zu trocknende Zwischenprodukte erhalten werden, wenn der Unterschuß an Formaldehyd 5 - 50 mol-%, bezogen auf die stechiometrisch einzusetzende Menge und vorzugsweise 5 - 20 mol-% beträgt. Im Gegensatz zum Stand der Technik wird jedoch nicht in einem organischen Lösungsmittel gearbeitet, welches mit dem Reaktionswasser azeotrop abdestilliert wird, sondern es wird Wasser zugesetzt. Diese Maßnahme führt überraschenderweise zu einer erhöhten Ausbeute an den letztendlich gewünschten monomeren 2-Cyanacrylaten.

Als Katalysatoren für die Umsetzung von Cyanessigestern mit Formaldehyd und/oder Polyoxymethylen können eine Vielzahl basischer Verbindungen und überraschend auch Säuren eingesetzt werden. Als basische Katalysatoren sind beispielsweise die in der Literatur bekannten sekundären oder tertiären Amine verwendbar. Es ist jedoch bevorzugt, Hydroxyde der Alkalimetalle oder Erdalkalimetalle einzusetzen. Die Verwendung dieser Verbindungen im erfindungsgemäßen Verfahren ist mit einer Reihe von

...

- 4 -

Vorteilen verknüpft. So sind Alkali- und Erdalkalimetallhydroxyde sehr wohlfeile Basen, welche zudem jederzeit verfügbar sind. Die Verbindungen sind weiterhin unter den Bedingungen des Crack-Verfahrens nicht flüchtig, so daß keine Verunreinigungen in die monomeren 2-Cyanacrylate eingeschleppt werden. Gerade die Verwendung flüchtiger Amine wie z.B. Piperidin    in dem Verfahren nach dem Stand der Technik bedingt eine Herabsetzung der Lagerfähigkeit der 2-Cyanacrylate, wenn nicht z.B. durch aufwendige Destillationstechniken oder Reinigungsoperationen ein Einschleppen des Katalysators in das Endprodukt verhindert wird.

Bei der Durchführung des erfindungsgemäßen Verfahrens können überraschenderweise auch saure Katalysatoren eingesetzt werden. Es hat sich gezeigt, daß bei pH-Werten zwischen 3 und 7, insbesondere aber zwischen 4 und 6 ähnliche Reaktionsgeschwindigkeiten wie bei der Basenkatalyse erreicht werden. Geeignete saure Katalysatoren sind z.B. Carbonsäuren aber auch Mineralsäuren, deren Salze und Mischungen, z.B. Pufferlösungen. Aus den obenstehend erörteten Gründen ist es in jedem Fall bevorzugt, Säuren einzusetzen, die unter Crack-Bedingungen nicht flüchtig sind und nicht in flüchtige Bestandteile zerfallen. Vorzugsweise werden deshalb Phosphorsäure oder Schwefelsäure bzw. deren saure Salze eingesetzt, da diese Säuren bekanntlich auch bei dem nachfolgenden Crack-Prozeß zugesetzt werden.

Die erfindungsgemäß einzusetzende Wassermenge kann in weiten Grenzen variiert werden. Es hat sich als günstig erwiesen, bezogen auf den Gesamtansatz 5 - 80 Gew.-% Wasser, vorzugsweise 10 - 50 und insbesondere 15 - 25 Gew.-%  Wasser einzusetzen. Hierbei bietet die

...

Wahl der Wassermenge ein einfaches Mittel den Reaktionsablauf zu steuern. So ist beispielsweise bei Wassermengen kleiner als 15 Gew.-% eine Steigerung der Reaktionstemperatur durch die Reaktionswärme bis zu etwa 100° und unter Druck sogar darüber möglich. Bei hohen Wassermengen, etwa 60 - 80 %, findet nur eine langsame Erwärmung auf etwa 40 - 80°C statt.

Nach einer                Ausführungsform der Erfindung werden Wasser und Formaldehyd gemeinsam eingesetzt. So können insbesondere im Handel erhältliche wässrige Formaldehydlösungen mit einem Gehalt an Formaldehyd zwischen 20 und 40 % als solche eingesetzt werden. Nach einer weiteren Ausführungsform werden wässrige Lösungen/Aufschlämmungen von Trioxan oder aber Aufschlämmungen von Polyoxymethylen (Paraformaldehyd) eingesetzt. Es können auch Mischungen der genannten Lösungen/Aufschlämmungen verwendet werden. So ist insbesondere die Verwendung nicht stabilisierter Formaldehydlösungen mit einem Anteil an Paraformaldehyd möglich. Zur Durchführung des Verfahrens werden die Cyanacetate im Reaktionsgefäß vorgelegt und Wasser sowie Formaldehyd bzw. Polyoxymethylen in den voraus berechneten Mengen zugegeben. Die Zugabe kann in einer oder in mehreren Portionen erfolgen. Durch die Reaktionswärme stellen sich Reaktionstemperaturen zwischen 40° und mehr als 130°C ein. Es ist bevorzugt, die Reaktion zwischen 80° und 120° durchzuführen. Hierbei kann nach einer speziellen Ausführungsform das Reaktionsgefäß verschlossen werden und unter dem Eigendruck des Gemisches eine Temperatur zwischen 100° und 120° gehalten werden. Nach einer weiteren Verfahrensvariante werden Wasser und Formaldehyd bzw. Polyoxymethylen in

. . .

- 6 -

zwei Portionen zum Reaktionsgemisch gegeben. Nach Zugabe der ersten Portion wird dabei das Reaktionsgefäß geschlossen und man läßt reagieren bis die Reaktortemperatur zu fallen beginnt. Die maximale Temperatur liegt zwischen 80 und 120°. Man läßt unter dem Siedepunkt des Wassers abkühlen, entspannt und gibt die zweite Portion Wasser und Polyoxymethylen zu. Es wird dann bei Temperaturen zwischen 100 und 120°, gegebenenfalls unter Kühlen oder Erwärmen belassen bis das Ende der exothermen Reaktion erreicht ist.

Nach der vorstehend geschilderten Umsetzung der Cyanacetate mit Formaldehyd oder Polyoxymethylen sieht das erfindungsgemäße Verfahren eine Entwässerungsstufe vor.

Diesewird vorzugsweise bei erhöhten Temperaturen z.B. zwischen 80 und 120° und unter vermindertem Druck durchgeführt.

Die so erhaltenen Produkte werden nach dem bekannten Crack-Verfahren in die monomeren 2-Cyanacrylate gespalten. Die 2-Cyanacrylate werden dabei in erhöhter Ausbeute und/oder nach Destillation in hoher Reinheit erhalten. Als Nebenprodukte entstehen Dicyandiglutarate, welche ihrerseits wieder zu 2-Cyanacrylate verarbeitet werden können. Die Verarbeitung der Dicyandiglutarate zu Cyanacrylate ist Gegenstand der deutschen Patentanmeldung ............... (eingereicht am gleichen Tag).

...

Nach dem erfindungsgemäßen Verfahren sind eine Vielzahl von monomeren 2-Cyanacrylaten in hoher Ausbeute und der für die Anwendung als Klebstoff erforderlichen Reinheit zugänglich. So können insbesondere hergestellt werden: Methyl-, Ethyl-, Propyl-, Isopropyl-, Butyl-, Isobutyl-, Hexyl-, Cyclohexyl-, 2-Ethylhexyl-, Benzyl-, Phenyl- und n-Decylester. Weiterhin können die Ester mit Glykolmonoethern als Alkoholkomponente, insbesondere der Ethylenglykol, Monomethyletherester bzw. der Ethylenglykolmonobutyletherester hergestellt werden.

## B e i s p i e l e

### Beispiel 1

In einem für Druckversuche ausgelegten Reaktionskessel werden 100 kg Cyanessigsäureethylester vorgelegt. Sodann werden 5 kg demineralisiertes Wasser, 12,75 kg Polyoxymethylen und 350 ml einer 30 %igen Natronlauge zugegeben. Der Kessel wird verschlossen und auf 60° erwärmt; es wird unter Ausnützung der Reaktionswärme eine maximale Reaktionstemperatur von 112° und ein Überdruck von 0,3 bar erreicht. Nach Ende der exothermen Reaktion wird entspannt, es werden weitere 15 kg demineralisiertes Wasser und 12,75 kg Polyoxymethylen zugegeben. Im geschlossenen Kessel bei einer Temperatur bis zu 118° und einem Überdruck von 0,3 bar läßt man sodann 40 Minuten weiter reagieren. Das zugegebene Wasser und das Reaktionswasser werden sodann abdestilliert, wobei zum Entwässern eine Dampfstrahlpumpe herangezogen wird. Nach Erreichen eines Restwassergehalts von 1000 ppm werden 500 g Phosphorsäure und 330 g Phosphorpentoxid zugegeben. Es wird dann die Spaltung des Zwischenproduktes bei Temperaturen zwischen 150 und 180° bei einem Druck kleiner 1 bar während 120 Minuten durchgeführt. Um Polymerisation zu vermeiden, wird dabei gasförmiges $SO_2$ durch das Reaktionsgemisch und das Destillat geleitet. Die thermische Spaltung lieferte 83,4 kg Rohester, aus denen durch erneute Destillation 50,5 kg Reinester hergestellt werden konnten. Der reine 2-Cyanacrylsäureethylester entsprach bezüglich Lagerstabilität, Abbindezeiten und zu erreichenden Zugscherfestigkeiten den anwendungstechnischen Anforderungen.

...

## Beispiel 2

Das erfindungsgemäße Verfahren wurde wiederholt. Dabei wurden 80,0 kg Cyanessigsäuremethylester vorgelegt und mit 2 x 12,2 kg Polyoxymethylen in Gegenwart von 2 x 8,0 kg Wasser und 270 ml 30 %iger Natronlauge zur Reaktion gebracht. Nach Trocknung und thermischer Spaltung wurden 50,0 kg Rohester erhalten, welche destillativ zu 40,0 g reinem 2-Cyanacrylsäuremethylester aufgearbeitet werden konnten.

## Beispiel 3

Beispiel 1 wurde wiederholt, jedoch wurde von 80,0 kg Cyanessigsäurebutylester ausgegangen. Es wurde mit 2 x 8,8 kg Polyoxymethylen in Gegenwart von 5,0 kg und anschließend 15,0 kg Wasser sowie 320 ml 30 %iger Natronlauge zur Reaktion gebracht. Nach Trocknung und thermischer Spaltung wurden 55,0 kg Rohester erhalten, die zu 36,5 kg reinem 2-Cyanacrylsäurebutylester aufarbeitbar waren.

## Vergleichsbeispiel

100 kg Cyanessigsäureethylester wurden in einem Reaktionskessel vorgelegt. Es wurden sodann 12,25 kg Polyoxymethylen, 9,5 kg Xylol sowie 419 ml einer 30 %igen Natriummethylatlösung in Methanol zugegeben. Nach Verschließen des Kessels wurde innerhalb von 15 Minuten eine Reaktionstemperatur von 116°C und ein Überdruck von 0,3 bar erreicht. Nach Abkühlen unter 100° wurden weitere 12,25 kg Polyoxymethylen (92 %ig) zugegeben und 40 Minuten bei einer Temperatur zwischen 95 und 115°C bei einem Überdruck bis zu 0,3 bar belassen. Während der Kondensations-

...

- 10-

reaktion destillierten bereits ca. 5,5 kg Xylol und ca. 8 kg Wasser ab. Vor der Trocknung wurden weitere 9,5 kg Xylol zugegeben und dann bei einer Temperatur von 115$^{\circ}$C und einem verminderten Druck (Wasserstrahlvakuum) 7,5 kg Wasser und der größte Teil des Xylols abdestilliert. In einer darauffolgenden Trockenstufe bei 115$^{\circ}$C und Dampfstrahlvakuum wurde ein Restwassergehalt kleiner 2000 ppm erreicht. Die analog ausgeführte thermische Spaltung lieferte 80,0 kg Rohester, welcher nach Destillation zu 42,1 kg reinem 2-Cyanacrylsäureethylester der geforderten Spezifikation aufarbeitbar waren.

...

Patentansprüche

1. Verfahren zur Herstellung von monomeren 2-Cyanacrylaten durch Umsetzung der entsprechenden Cyanacetate mit einem molaren Unterschuß an Formaldehyd und/oder Polyoxymethylen bei erhöhten Temperaturen unter anschließender thermischer Spaltung des entstandenen Zwischenproduktes, dadurch gekennzeichnet, daß man die Umsetzung der Cyanacetate mit Formaldehyd und/oder Polyoxymethylen in Gegenwart von Wasser bei einem pH-Wert von 3 - 7 oder 7 - 14, gegebenenfalls unter erhöhtem Druck durchführt.

2. Verfahren nach Anspruch 1, dadurch gekennzeichnet, daß ein pH-Wert größer 7 mit Alkali- und/oder Erdalkalimetallhydroxyden eingestellt wird.

3. Verfahren nach Anspruch 1, dadurch gekennzeichnet, daß ein pH-Wert kleiner gleich 7 mit schwer flüchtigen Säuren, insbesondere mit Schwefelsäure, Phosphorsäure und/oder deren partiellen Salzen eingestellt wird.

4. Verfahren nach den Ansprüchen 1 - 3, dadurch gekennzeichnet, daß man Wasser in Mengen von 5 - 80 Gew.-%, bezogen auf Gesamtansatz, vorzugsweise in Mengen von 10 - 50 Gew.-% und insbesondere von 15 - 25 Gew.-% einsetzt.

5. Verfahren nach den Ansprüchen 1 - 4, dadurch gekennzeichnet, daß man die Cyanacetate vorlegt und das Wasser sowie die vorausberechnete Menge an Formaldehyd und/oder Polyoxymethylen auf einmal oder portionsweise zugibt, wobei nach jeder Zugabe das Reaktionsgefäß

...

- 12 -

verschlossen wird und unter Ausnützung der Reaktionswärme eine Temperatur zwischen 80 und 120$^{O}$C eingestellt wird.